# EUROPEAN PATENT APPLICATION

(11) **EP 2 264 150 A1**
(43) Date of publication of application: **22.12.2010**
(21) Application number: 09162256.3
(22) Date of filing: 09.06.2009
(51) Int. Cl.: C12N 5/078, A61K 35/14, A61M 1/36, B01D 21/26, A61P 19/00

(54) **Triple spin, double pool and revolumization process for concentrating platelets and derivative platelets concentrate**

(71) Applicant: Smith, Astley E., Vancouver, British Columbia V6N 3M9 (CA)
(72) Inventor: Smith, Astley E., Vancouver, British Columbia V6N 3M9 (CA)
(74) Representative: Evans, Huw David Duncan

(57) **Abstract**

The present invention comprises an improved method of manufacturing platelet concentrate comprising the steps of: performing a first centrifugation of anticoagulated blood, pooling a first platelet rich sample, performing a second centrifugation on the first platelet rich sample whereby the volume of the first platelet rich sample placed in each subsequent centrifuge container is greater than the extracted volume from each previously utilized container, pooling a second platelet rich sample, performing a third centrifugation on the second platelet rich sample, whereby the volume of the second platelet rich sample placed in each subsequent centrifuge container is greater than the previous extracted volume from each previously utilized container, decanting a portion of supernatant overlying a third platelet rich sample, and suspending the third platelet rich sample such as to form a highly concentrated platelet rich formulation at concentrations greater than 8 times and up to 60 times baseline.

## Description

### 1. FIELD OF THE INVENTION:

The present invention relates to improved methods of manufacture of highly concentrated platelet formulations, for use in wound healing and bone regeneration, and in particular dental healing and bone regeneration.

### 2. BACKGROUND OF THE INVENTION:

Platelets or thrombocytes are blood cells that play a critical role in healing and the generation of new tissue. Platelets promote the formation of blood clots that act as a physical barrier at a site of rupture of a blood vessel, thereby preventing infection and avoiding further blood loss. Additionally, platelets have the capacity to stimulate angiogenesis (formation of new blood vessels) and increase collagen formation, cell division and cell growth.

Enriched platelet concentrates are known to possess advantages in healing and formation of new tissue. The use of platelet rich plasma (PRP) in surgical sites has been shown to rapidly produce both hard and soft tissue regeneration and repair. Whitman et al. 1997. Platelet gel: An autologous alternative to fibrin glue with applications in oral and maxillofacial surgery. J. Maxillofac Surg. 55:1294-9. In one example, a linear relationship was demonstrated between release of growth factor PDGF-AB and the number of platelets, where the highest concentration of platelets used was 1.6 million/µl or approximately 8 times baseline. Marx, Robert E. and Garg, Arun K. 2005. Dental and Craniofacial Application of Platelet-Rich Plasma. Quintessence Publishing Co. Inc. 31-47, 53-75.

Consequently, it is desirable to produce platelet concentrates, such as PRP, having elevated platelet concentrations. PRP is commonly produced by centrifuging whole blood to separate a supernatant of platelet-containing plasma from the remainder of the blood components; centrifuging the supernatant to separate platelet poor plasma (PPP) from a precipitate or pellet of highly concentrated platelets; and re-suspending the pellet in a small amount of plasma or other approved solution to form a concentrated PRP or other platelet concentrate To date, automated centrifuges and other centrifugation techniques have been commonly used to carry out the extraction and concentration of platelets, but such techniques have been limited in their capacity to concentrate platelets, and have produced, at best, concentrates having platelet concentrations of approximately 8 times baseline, with peak platelet concentrations of about 4400 x 10³/µl. G. Weibrich et al. Curasan PRP kit vs. PCCS PRP System. Clin. Oral Impl. Res. 13: 2002/437 - 443.

There is a requirement in the art of dental and other wound healing and bone regeneration for improved platelet concentrate formulations to further accelerate wound healing and bone regeneration.

### 3. SUMMARY OF THE INVENTION

The present invention involves highly concentrated platelet formulations, including greater than 8 times baseline, preferably approximately 23 times to 45 times baseline, and optimally up to approximately 60 times baseline (where baseline is defined as the initial concentration of platelets in a sample of whole blood). Platelet concentrate solutions of approximately 23 times baseline have been shown by the applicant in particular to yield markedly superior results to the current art in dental wound healing and bone regeneration. The attainment of such highly concentrated platelet samples has not been achievable by conventional techniques.

In the manufacture of the above highly concentrated platelet formulations, the present invention employs a novel series of centrifugation steps in combination with the strategic pooling and revolumization of platelet rich substrates to produce a highly concentrated platelet concentrate (hcPC) or highly concentrated platelet rich plasma (hcPRP) having a platelet concentration greater than 8 times baseline, and commonly in the range of approximately 23 times baseline to 45 times baseline, and up to approximately 60 times baseline.

Another aspect of the invention is the concentration of platelets by means of a novel serial centrifugation, strategic pooling and revolumization process, whereby platelet rich samples isolated from one centrifuge step (whether in a supernatant or in a resuspension) are strategically pooled, and redistributed (or "revolumized") such that the volume of the extracted platelet rich sample from a prior centrifuge container is increased within its subsequent centrifuge container prior to a subsequent centrifugation step.

For clinical practicality, such as for use in a dental office, a first centrifugation (first spin) of whole blood followed by a first collection, strategic pooling and revolumization (first pooling and revolumization) of platelet rich supernatant; thereafter a second centrifugation (second spin) followed by a second collection, strategic pooling and revolumization (second pooling and revolumization) of platelet rich precipitate in suspension; and thereafter a third centrifugation (triple spin) followed by a decantation of platelet poor supernatant overlying a highly concentrated platelet rich precipitate, yields a highly concentrated grouping of platelets. The further step of re-suspending the platelet rich precipitate in a small remainder of the overlying platelet poor supernatant gives rise to hcPRP having a platelet concentration of greater than 8 times baseline, and commonly approximately 23 to 45 times baseline, and up to approximately 60 times baseline. In conversion to absolute concentrations, platelet concentrations of hcPRP of greater than 4800 x 10³/µl - 10000 x 10³ /µl, and optimally up to approximately 15000 x 10³/µl are achievable by the current inventive process, which is of greater concentration as compared to what has been achievable in the prior art.

In accordance with the present invention, the essence of the disclosed process for concentrating platelets can be summarized in the following general steps:
a. performing a first centrifugation on an anticoagulated sample of blood;
b. extracting and pooling a first platelet rich sample;
c. performing a second centrifugation on said first platelet rich sample, whereby the average volume of said first platelet rich sample placed in each subsequent centrifuge container is greater than the average extracted volume from each previously utilized centrifuge container;
d. withdrawing and pooling a second platelet rich sample;
e. performing a third centrifugation on said second platelet rich sample, whereby the average volume of said second platelet rich sample placed in each subsequent centrifuge container is greater than the average previous extracted volume from each previously utilized centrifuge container; and
f. decanting at least part of a supernatant overlying a third highly concentrated platelet rich sample.

The final step of suspending the third highly concentrated platelet rich sample, which is embodied as a pellet, with preferably a small portion of the overlying supernatant atop of the third highly concentrated platelet rich sample forms hcPRP at concentrations of greater than 8 times baseline, commonly in the range of approximately 23 to 45 times baseline, and up to approximately 60 times baseline.

### 4. BRIEF DESCRIPTION OF THE DRAWINGS

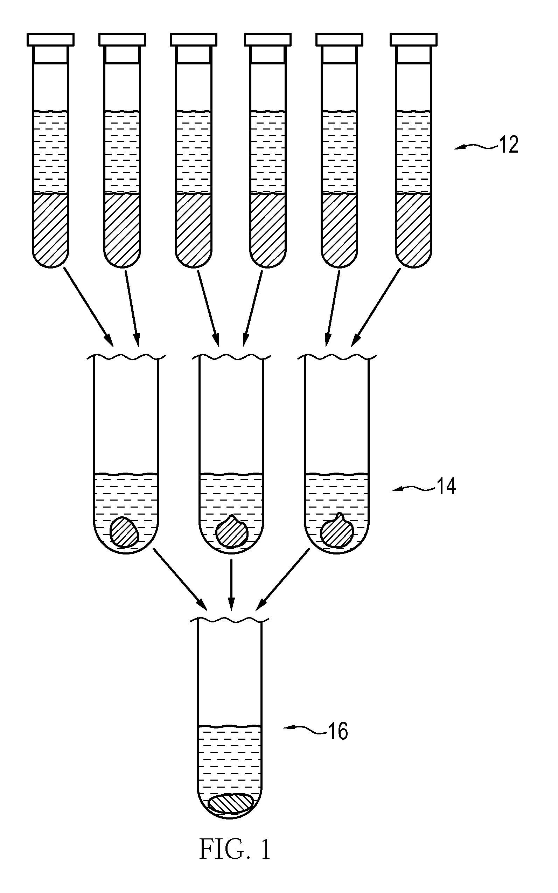

Figure 1 is a side elevation view of a number of first, second and third containers following centrifugation, and depicts the transfer of platelet rich plasma from the first containers through to the third container.

### 5. DETAILED DESCRIPTION AND PREFERRED EMBODIMENT

The present invention relates to a novel, highly concentrated platelet formulation and method of manufacturing the same, in view of using such formulation in the promotion or acceleration of wound healing and bone regeneration, preferably in the field of dental surgery.

The following details the preferred method according to the inventor. It will be understood that some of the steps may be practiced in a different order from which they are set out below. The order in which the steps appear in this specification is not necessarily the order in which they must be performed but indicates the preferred order.

In accordance with the preferred embodiment of the present invention, there is provided a platelet concentrate having a novel platelet concentration of greater than 8 times baseline, and commonly in the range of approximately 23 times baseline to 45 times baseline, and up to approximately 60 times baseline, as manufactured by the following process:
a. placing a sample of whole blood into a first plurality of containers;
b. adding an anticoagulant to at least part of said sample at any point prior to initiation of step c;
c. performing a first centrifugation of said sample within said first plurality of containers;
d. decanting a first supernatant of platelet rich plasma from each of said first plurality of containers, following said first centrifugation;
e. performing a first pooling of said first supernatant;
f. placing said first supernatant once pooled into a second plurality of containers;
g. performing a second centrifugation on said first supernatant within said second plurality of containers, such as to form a second supernatant and a first precipitate within each of said second plurality of containers;
h. decanting at least a portion of said second supernatant from each of said second plurality of containers;
i. forming a resuspension of said first precipitate;
j. performing a second pooling of said resuspension into at least one container;
k. performing a third centrifugation on said resuspension within said at least one container, such as to form a third supernatant, and a second precipitate; and
l. decanting at least a portion of said third supernatant;
   wherein the average volume of said first supernatant within each of said first plurality of containers is less than the average volume of said first supernatant within each of said second plurality of containers, and wherein the average volume of said re-suspension within each of said second plurality of containers is less than the average volume of said re-suspension within said at least one container; and
   whereby said second precipitate comprises a highly concentrated platelet concentrate (hcPC).

The creation of hcPRP of concentrations of greater than 8 times baseline, commonly in the range of approximately 23 times baseline to 45 times baseline and up to approximately 60 times baseline is accomplished by a final step of preferably suspending the second precipitate into a small amount of the third overlying supernatant or another suitable solution approved by regulatory authorities. hcPRP concentrations of greater than 4800 x 10³/µl - 10000 x 10³ /µl, and up to approximately 15000 x 10³/µl, assuming a typical initial baseline concentration of approximately 220 x 10³/µl, are readily achievable by the disclosed process. Optionally, if a large enough sample of whole blood is initially collected, a more concentrated sample of hcPRP can be obtained wherein the final pellet of hcPC is mixed to form a suspension with a portion of the first supernatant of platelet rich plasma, which may be saved from step (d) above.

In a particular example of the above method with prescribed volumes, spin speeds and centrifugation durations included, the process preferably consists of the following steps:
(i) collecting approximately 60 - 120 ml of whole blood treated with an anticoagulant and dividing it in approximately equal amounts between 4 and 10 and most preferably 6 centrifuge-adapted first containers;
(ii) centrifuging each first container at a speed consistent with approximately 100 to 140 g, and most preferably 120g, for approximately 8 to 12 minutes, most preferably 10 minutes to separate from the blood a first supernatant;
(iii) decanting the first supernatant from each first container into a number of second, centrifuge-adapted containers that is less, and preferably half the number of the first containers (preferably 3), so that each second container has approximately an equal volume of first supernatant;
(iv) centrifuging each second container at a speed consistent with approximately 700 to 1100 g, most preferably 900 g for approximately 8 to 12 minutes, most preferably 10 minutes to separate the first supernatant into a second supernatant and a first platelet pellet;
(v) decanting the second supernatant from each second container such that approximately 1.0 - 2.0, preferably approximately 1.5 ml of the second supernatant remains therein;
(vi) re-suspending in each second container the first platelet pellet in the second supernatant to form a first platelet rich plasma;
(vii) transferring the first platelet rich plasma from each second container to a number of third, centrifuge-adapted containers (preferably 1);
(viii) centrifuging the third container at a speed consistent with approximately 200 to 600g, most preferably 400g for approximately 8 to 12 minutes and preferably for 10 minutes to separate the first platelet rich plasma into a third supernatant and a second platelet pellet;
(ix) decanting the third supernatant from the third container such that up to approximately 1.0 - 3ml, preferably 2 ml of the third supernatant and the second platelet pellet remain; and
(x) suspending the second platelet pellet in an amount of approved solution, preferably approximately 1.0 - 3 ml, preferably 2 ml of the remaining third supernatant, to form the second, most highly concentrated platelet rich plasma (hcPRP).

The invention provides a platelet concentrate or platelet rich plasma that has a baseline platelet concentration greater than 8 times baseline, commonly approximately 23 to 45 times baseline, and up to approximately 60 times baseline, which is a concentration higher than that found in platelet concentrates or platelet rich plasma of the prior art.

Platelet concentrate, hcPC or hcPRP of a desired concentration may thereby be produced by obtaining a prescribed volume of anticoagulant-treated whole blood and, in accordance with the select method, centrifuging the blood and derivative platelet rich concentrate at the prescribed speed and duration and pooling and re-adjusting (or increasing) the volume of the derivative platelet rich concentrate within subsequent centrifuge tubes (or containers) on each re-centrifugation step.

The steps of manufacture of hcPC or hcPRP as disclosed to the present invention preferably occurs in a dental or other equivalent medical office, wherein the initial whole blood sample is collected from the patient ("autologously") at the time or just prior to dental surgery in which the patient will require superior dental wound healing. The product hcPRP is particularly useful in the promotion or acceleration of osteogenesis, a vital component of post dental surgical healing, and more particularly in implant surgery.

The hcPRP of the present invention is osteoconductive, osteoinductive, osteogenic and likely chondrogenic. New bone is formed from the hcPRP in individuals when the hcPRP, preferably having a platelet concentration of approximately 23 times baseline, is placed at a surgical site, preferably the sinus. Insertion or grafting of bone material is thereby avoided.

In one example, whole blood is obtained having a typical baseline platelet concentration of approximately 213 x 10³/µl. Commencing with a typical blood volume of 60 ml, re-suspending the second platelet pellet in approximately 2 ml of third supernatant provides a platelet rich plasma with a platelet concentration of greater than 4800 x 10³/µl, which represents an approximately twenty-three-fold increase in platelet concentration.

Figure 1 illustrates centrifugation of first (12), second (14) and third (16) containers and pooling of platelet rich plasma from the first through to the third containers in accordance with the invention. The preferred centrifuge is an FDA approved manual centrifuge. In the preferred embodiment, the centrifuge accepts at least 6 containers at a time. The preferred first, second and third containers are Vacutainers^{®} or similar containers designed for centrifugation.

In an example of use of the present invention, a number of first containers of equal size are selected, preferably 6 as depicted in Figure 1. A volume of whole blood, preferably 60 ml, is distributed between the first containers such that each first container holds an approximately equal volume of blood. The blood must be treated prior to centrifugation with an anticoagulant, preferably sodium citrate, to reduce aggregation of the platelets. The anticoagulant may be added to the blood before or after the blood has been transferred to the first containers.

All the first containers are then simultaneously centrifuged at a prescribed speed and duration, preferably at approximately 120g for approximately 10 min. This separates the heavier components of the blood, such as red blood cells, from the platelet rich plasma, which forms an upper layer or first supernatant that is distinct from the heavier components in the sediment layer. At this stage, the platelets remain suspended in the plasma of the first supernatant.

Following completion of the first centrifugation, the first supernatant in each first container is decanted or removed from the sediment layer and pooled into a number of second containers (of preferably equal size to the first), that is half the number of the first containers, preferably three (3). Decanting may be carried out with the aid of a pipette or other suitable instrument. Following decanting, each second container holds an approximately equal portion of the total volume of the first supernatant derived from the first containers.

All the second containers are simultaneously centrifuged at a prescribed speed and duration, preferably at approximately 900g for approximately 10 min. In the preferred embodiment, a counterweight is used to balance the three (3) second containers prior to commencing centrifugation. The second centrifugation causes formation in each second container of a first platelet pellet and a second supernatant of platelet-poor plasma.

Following completion of the second centrifugation, the second supernatant in each second container is decanted by a pipette or other suitable means, such that a reduced volume of second supernatant remains. In the preferred embodiment, approximately 1.5 ml of the second supernatant remains in each second container following decanting. The first platelet pellet in each second container in then resuspended in the second supernatant remaining therein, thereby forming a first platelet rich plasma. Re-suspension is preferably carried out by aspiration and ejection repeated three times. The first platelet rich plasma in each second container is then pooled into a single third container, which for ease of convenience is of equal size to the other containers. In the preferred embodiment, this results in approximately 4.5 ml of first platelet rich plasma in the third container.

The third container is then centrifuged at a prescribed speed and duration, preferably at approximately 400g for approximately 10 min. In the preferred embodiment, a counterweight is used to balance the third container prior to commencing centrifugation. The third centrifugation causes formation in the third container of a second platelet pellet and a third supernatant of platelet-poor plasma.

Following completion of the third centrifugation, the third supernatant in the third container is decanted by a pipette or other suitable means, such that a reduced volume of third supernatant remains. In the preferred embodiment, approximately 2 ml of the third supernatant remains following decanting.

The second platelet pellet is next suspended in an appropriate approved solution of a desired volume to result in a platelet concentrate or platelet rich plasma with a desired platelet concentration of approximately 23 times baseline to 45 times baseline, and up to approximately 60 times baseline. Preferably, the second platelet pellet is resuspended by aspiration and ejection repeated three times in approximately 2ml of the third supernatant remaining in the third container, so as to form hcPRP of greater than 8 times baseline, commonly in the range of approximately 23 times baseline to 45 times baseline, and up to approximately 60 times baseline. The resulting hcPC or hcPRP is then used for any purposes as are known in the art.

Preferably, the anticoagulant within the hcPRP is reversed, and thrombin (preferably autologously derived or optionally derived from an animal donor) is utilized to activate the hcPRP, which is thereafter ready for dental surgical application. The activated hcPRP is then preferably applied to a carrier, such as a re-absorbable collagen sponge, which can applied and retained on a surgical wound site.

The present invention also relates to kits comprising materials and instructions necessary to practice the present methods. Preferred kits include ones comprising instructions for practicing a method in accordance with the present invention, along with a blood collection kit, a centrifuge, or both. A manual centrifuge is preferred, optimally with 6 rotor positions. Preferably, the centrifuge has variable speed. A set of re-absorbable collagen sponges may be included. Preferably, the above mentioned instructions take the form of a hcPRP instruction manual.

While the embodiment disclosed comprises a preferred method of manufacture of platelet concentrate performed at the time of a dental surgery, within or directly proximate to a dentist's office, and with an autologously obtained blood sample taken from a patient, alternatively, it is also possible to concentrate non-autologously (or "homologously") obtained donor platelets from a remote location by the disclosed methods of the present invention. In this latter alternative embodiment, a dentist or other surgeon would purchase donor platelet concentrate and have it shipped from the remote location, which may, for example, comprise an independent blood collection and processing unit. In this alternative method, a patient in need of platelet concentrate would have their blood typed, and the appropriate donor hcPC, hcPRP, or other highly concentrated platelet concentrate formulation would be obtained and stored prior to a planned dental procedure.

With a larger input sample of whole blood available, as described earlier, it is also possible to further concentrate product hcPRP by utilizing saved PRP from a previous centrifugation step, as opposed to utilizing PPP derived from the final centrifugation step (or other approved non-platelet rich solution), for the final step of re-suspending the hcPC pellet to form a useful suspension.

Furthermore, while the preferred embodiment to the present invention discloses a method of manufacturing platelet concentrate performed manually by a medical practitioner or lab assistant in conjunction with a centrifuge and blood collection kit (including the manual steps of extracting supernatant, pooling and revolumizing, and suspending supernatant or other approved solution to form a suspension), alternatively the disclosed methods could be fully automated by a stand alone centrifuge system, which enables the aforementioned triple spin, double pool, and revolumization process (to form hcPC), and optionally the final re-suspension process (to form hcPRP, or other highly concentrated platelet solution), without substantial need of further intervention of a human medical practitioner or equivalent thereof.

In this alternative embodiment of automated centrifugation, a centrifuge would be adapted to receive an anti-coagulated blood sample from an operator, and then perform independently any of the aforementioned steps, enabling formation of hcPC and optionally hcPRP (or other equivalent platelet rich solution), after which, the desired platelet rich sample product would be collected by an operator.

While specific embodiments of the invention have been described and illustrated, such embodiments should be considered illustrative of the invention only, and not as limiting the invention as construed in accordance with the accompanying claims.

## Claims

1. A method of producing platelet concentrate, comprising the steps of:
a. performing a first centrifugation on an anticoagulated sample of blood;
b. withdrawing and pooling a first platelet rich sample;
c. performing a second centrifugation on said first platelet rich sample, whereby the volume of said first platelet rich sample in each centrifuge container utilized is greater than the previous extracted volume;
d. withdrawing and pooling a second platelet rich sample;
e. performing a third centrifugation on said second platelet rich sample, whereby the volume of said second platelet rich sample in each centrifuge container utilized is greater than the previous extracted volume; and
f. decanting at least part of a supernatant overlying a third highly concentrated platelet rich sample.

2. The method according to claim 1, additionally comprising the initial steps of:
i). placing a sample of whole blood into a first plurality of containers; and
ii). adding an anticoagulant to at least part of said sample of whole blood at any
point prior to initiation of step (a) of claim 1, to form an anticoagulated sample of blood.

3. The method according to any one of claims 1 - 2, wherein said withdrawing and pooling a second platelet rich sample additionally comprises forming a re-suspension of said second platelet rich sample.

4. The method according to claim 3, wherein said withdrawing and pooling a second platelet rich sample comprises withdrawing a second supernatant solution, and wherein said re-suspension of said second platelet rich sample is accomplished by suspending said second platelet rich sample with a remainder of said second supernatant solution,

5. The method according to any one of claims 2 - 4, wherein said anticoagulant comprises sodium citrate.

6. The method according to any one of claims 3 - 5, wherein said re-suspension has a platelet concentration in the range of at least one of:
greater than 8 times to approximately 60 times the baseline platelet concentration of said sample of whole blood of step 2 (i); and
greater than 4800 x 10³/µl to approximately 15000 x 10³/µl.

7. The method according to any one of claims 1 - 6, wherein steps 1 (a) through (f) are performed automatically by a centrifuge.

8. The method according to any one of claims 1 - 7, wherein said second centrifugation comprises fewer centrifuge containers than said first centrifugation, and said third centrifugation comprises fewer centrifuge containers than said second centrifugation.

9. The method according to any one of claims 1 - 8, said method further comprising the step of re-suspending said third highly concentrated platelet rich sample by re-suspending said third highly concentrated platelet rich sample using a portion of said supernatant such as to produce platelet rich plasma having a platelet concentration in the range of at least one of:
greater than 4800 x 10³/µl to approximately 7500 x 10³/µl;
approximately 7500 x 10³/µl to approximately 10000 x 10³/µl;
approximately 10000 x 10³/µl to approximately 12500 x 10³/µl;
approximately 12500 x 10³/µl - approximately 15000 x 10³/µl;
approximately 23 to 30 times baseline;
approximately 30 to 40 times baseline;
approximately 40 to 50 times baseline; and
approximately 50 to 60 times baseline.

10. A platelet formulation produced by the method according to any one of claims 1 - 9, said method additionally comprising the step of:
g. suspending said third highly concentrated platelet rich sample in a
solution;
whereby said third highly concentrated platelet rich sample suspended in said solution comprises said platelet formulation; and
wherein said platelet formulation has a platelet concentration in the range of at least one of:
greater than 4800 x 10³/µl to approximately 15000 x 10³/µl;
approximately 7500 x 10³/µl to approximately 10000 x 10³/µl;
approximately 10000 x 10³/µl to approximately 12500 x 10³/µl;
approximately 12500 x 10³/µl to approximately 15000 x 10³/µl;
approximately 12500 x 10³/µl to approximately 15000 x 10³/µl;
approximately 23 to 30 times baseline;
approximately 30 to 37 times baseline;
approximately 37 to 45 times baseline;
approximately 45 to 53 times baseline; and
approximately 53 to 60 times baseline.

11. A formulation of platelet concentration in the range of at least one of: greater than 8 times baseline to approximately 60 times baseline, and greater than 4800 x 10³/µl to approximately 15000 x 10³/µl, said formulation produced by the following method:
a. inputing a sample of whole blood into a first plurality of containers;
b. adding an anticoagulant to at least part of said sample at any point prior to initiation of step 11 (c);
c. performing a first centrifugation of said sample within said first plurality of containers;
d. decanting a first supernatant of platelet rich plasma from each of said first plurality of containers, following said first centrifugation;
e. performing a first pooling of said first supernatant;
f. placing said first supernatant once pooled into a second plurality of containers;
g. performing a second centrifugation on said first supernatant within said second plurality of containers, such as to form a second supernatant and a first precipitate within each of said second plurality of containers;
h. decanting at least a portion of said second supernatant from each of said second plurality of containers;
i. forming a first re-suspension of said first precipitate;
j. performing a second pooling of said re-suspension into at least one container;
k. performing a third centrifugation on said first re-suspension within said at least one container, such as to form a third supernatant, and a second precipitate;
l. decanting at least a portion of said third supernatant; and
m. forming a second re-suspension of said second precipitate by suspending
said second precipitate with a solution to form said formulation;
whereby said second re-suspension has a platelet concentration in the range of at least one of: greater than 8 times baseline to approximately 60 times the baseline platelet concentration of said sample of whole blood of step 28 (a), and greater than 4800 x 10³/µl to approximately 15000 x 10³/µl.

12. The formulation according to claim 11, wherein the average volume of said first supernatant within each of said first plurality of containers is less than the average volume of said first supernatant within each of said second plurality of containers, and wherein the average volume of said first re-suspension within each of said second plurality of containers is less than the average volume of said first resuspension within said at least one container.

13. The formulation according to any one of claims 11 and 12 for use in promoting osteogenesis, said formulation having a platelet concentration in the range of at least one of: greater than 4800 x 10³/µl to approximately 15000 x 10³/µl, and approximately 23 times baseline to approximately 60 times baseline.

14. A kit for use in concentrating platelets for wound healing, said kit comprising:
a. A centrifuge; and
b. A set of instructions for use of said centrifuge, in practice of the method according to any one of claims 1 - 9.

15. The kit according to claim 14, further comprising at least one of: a blood collection kit, and a set of re-absorbable collagen sponges.
